# EUROPÄISCHE PATENTSCHRIFT

(11) **EP 1 536 232 B1**
(45) Veröffentlichungstag und Bekanntmachung des Hinweises auf die Patenterteilung: **10.11.2010**
(21) Anmeldenummer: 04027831.9
(22) Anmeldetag: 24.11.2004
(51) Int. Cl.: G01N 33/577, G01N 33/74, G01N 33/68

(54) **Analytischer Sandwichtest zur Bestimmung von NT-proBNP**
Analytical sandwich assay for the determination of NT-proBNP
Analyse en essaie de sandwich pour la détermination du peptide NT-proBNP

(30) Priorität: 28.11.2003 DE 10355731
(43) Veröffentlichungstag der Anmeldung: 01.06.2005
(73) Patentinhaber: Roche Diagnostics GmbH, 68305 Mannheim (DE); F.Hoffmann-La Roche AG, 4070 Basel (CH)
(72) Erfinder: Spinke, Juergen, Dr., 64653 Lorsch (DE); Nichtl, Alfons, Dr., 82383 Hohenpeissenberg (DE); Klemt, Volker, Dr., 82362 Weilheim (DE); Hallermayer, Klaus, Dr., 82340 Feldafing (DE); Grol, Michael, Dr., 82340 Feldafing (DE); Borgya, Anneliese, 82402 Seeshaupt (DE); Gallusser, Andreas, Dr., 82377 Penzberg (DE)

(56) Entgegenhaltungen:
- EP-A1- 0 186 799
- WO-A-00/35951
- WO-A-2004/046194
- WO-A-2004/099252
- WO-A-2004/099253
- WO-A2-00/45176
- SHIMIZU HIROYUKI ET AL: "Characterization of molecular forms of probrain natriuretic peptide in human plasma." CLINICA CHIMICA ACTA, Bd. 334, Nr. 1-2, August 2003 (2003-08), Seiten 233-239, XP002381702 ISSN: 0009-8981
- KARL J ET AL: "DEVELOPMENT OF A NOVEL, N-TERMINAL-PROBNP (NT-PROBNP) ASSAY WITH A LOW DETECTION LIMIT" SCANDINAVIAN JOURNAL OF CLINICAL AND LABORATORY INVESTIGATION, BLACKWELL SCIENTIFIC PUBLICATIONS, OXFORD, GB, Bd. 59, Nr. SUPPL 230, 1999, Seiten 177-181, XP009045882 ISSN: 0085-591X

## Beschreibung

Die vorliegende Erfindung betrifft einen analytischen Sandwichtest, insbesondere ein Testelement, insbesondere in Form eines nach dem Sandwich-Prinzip arbeitenden immunochromatographischen Teststreifens, für die Bestimmung von N-terminalem pro-Brain Natriuretic Peptide (NT-proBNP).

NT-proBNP ist ein viel versprechender Marker für die Diagnose und das Management von Herzinsuffizienz. Eine Übersicht über Herzinsuffizienz und die Bedeutung von NT-proBNP als Markersubstanz hierfür findet sich beispielsweise in WO 00/45176 auf den Seiten 1 bis 4. Weiterhin sind die Dokumente WO 00/35951, US 5,786,163, US 6,461,828, US 6,117,644, EP 1 151 304, WO 02/083913 und die EP-Patentanmeldung Nr. 03 010 591.0 vom 12.5.2003 (Klemt et al.) veröffentlicht als WO 2004/099252 und WO 2004/099253 mit NT-proBNP, Antikörpern hierzu und Bestimmungsverfahren hierzu befasst.

Der einzige zur Zeit im In-vitro-Diagnostik-Markt verfügbare NT-proBNP-Test ist der vollautomatisierte Elecsys® NT-proBNP-Test von Roche Diagnostics, welcher auf Basis einer Sandwichreaktion mit Elektrochemilumineszenzdetektion arbeitet. Dieser Test ist für den Einsatz in großen Zentrallabors konzipiert und benötigt zur Durchführung neben flüssigen, genau zu dosierenden Reagenzien ein verhältnismäßig komplexes Gerät zur Dosierung der Flüssigkeiten und zur Detektion des Lumineszenzsignals. Ein einfach zu bedienender, zur Not visuell ohne Auswertegerät auskommender Schnelltest für NT-proBNP ist zur Zeit nicht auf dem Markt.

Schnelltests für immunologisch nachweisbare Substanzen sind für eine Vielzahl von unterschiedlichen Parametern seit langem bekannt, beispielsweise aus WO 97/06439, EP 0 291 194, US 5,591,645, US 4,861,711, US 5,141,850, US 6,506,612, US 5,458,852, US 5,073,484. Hier werden meist die immunolgischen Nachweisreagenzien (im Wesentlichen markierte und unmarkierte Antikörper bzw. Antigene) in trockener Form auf einem Träger bereitgestellt, der den Transport einer Probenflüssigkeit (insbesondere Körperflüssigkeiten wie Blut, Serum, Plasma, Urin, Speichel etc.) auf oder in dem Träger erlaubt. Vorzugsweise ist der Träger hierzu kapillaraktiv, beipielsweise eine Membran oder ein mit Kapillarkanälen versehener Kunststoffträger. In der Fachwelt spricht man oft von immunchromatographischen Teststreifen oder Testdevices.

Bei Patienten mit akuter Atemnot ist es von Vorteil so schnell wie möglich eine NT-proBNP-Bestimmung durchzuführen um eine Herzinsuffizienz als Ursache der Atemnot auszuschliessen bzw. zu diagnostizieren und eine entsprechende Behandlung einzuleiten. Da der Elecsys® NT-proBNP-Test nur im Zentrallabor durchgeführt werden kann, ist eine schnelle NT-proBNP-Bestimmung ausserhalb der Routinezeiten nur schwer möglich. Es wäre daher gerade für die Notfallstation von Vorteil wenn ein Schnelltest zur Verfügung stünde, der auch ausserhalb der Routinezeiten direkt in der Notfallstation durchgeführt werden könnte. Der Schnelltest sollte dabei aber die gleichen Referenzbereiche und cut-offs gewähren wie die Referenzmethode im Zentrallabor (Elecsys® NT-proBNP), um so eine gute Vergleichbarkeit der Ergebnisse - unabhängig von der tatsächlich durchgeführten Art des Tests - zu ermöglichen.

Die im Elecsys® NT-proBNP-Test verwendeten polyklonalen Antikörper (PAK) erkennen eine ganz spezielle Fraktion des NT-proBNP ("natives" NT-proBNP; siehe EP-Patentanmeldung Nr. 03 010 591.0 vom 12.5.2003 von Klemt et al.; erkannt werden demnach die Epitope von NT-proBNP mit den Aminosäuren 1-21 (AS 1-21) und 39-50 (AS 39-50)). Es hat sich jedoch gezeigt, dass diese polyklonalen Antikörper für NT-proBNP-Schnelltests, die partikuläre Label wie z. B. kolloidales Gold als Label verwenden, ungeeignet sind, da sie auf Grund physiko-chemischer Wechselwirkungen mit den Schnelltestkomponenten (wie z. B. Trägermaterialien, Matrizes etc.) eine hohe, unerwünschte Variabilität in der Signalgenerierung zeigen. Dies hat deutliche Qualitätsschwankungen des Testes von Charge zu Charge zur Folge.

Aufgabe der vorliegenden Erfindung war es deshalb, die Nachteile des Standes der Technik zu beseitigen. Insbesondere sollte ein reproduzierbar herstellbarer und mit guter Korrelation zur Labormethode ausgestatteter Schnelltest für die Bestimmung von NT-proBNP bereitgestellt werden.

Diese Aufgabe wird durch den Gegenstand der Erfindung gelöst.

Gegestand der Erfindung ist ein immunologischer Test, insbesondere in Form eines Schnelltests wie z. B.eines analytischen Testelements, wie er in den unabhängigen Patentansprüchen charakterisiert ist. Bevorzugte Ausführungsformen sind in den abhängigen Ansprüchen wiedergegeben.

Die erfindungsgemässe Lösung zur Herstellung eines immunologischen Tests im Sandwich-Format, insbesondere eines Schnelltestes, der gut zur Elecsys® Referenzmethode korreliert, sieht die Verwendung einer speziellen Antikörperkombination umfassend mindestens zwei Antikörper gegen NT-proBNP vor, wobei mindestens ein Antikörper ein monoklonaler Antikörper (MAK; im Englischen: monoclonal antibody oder MAB) ist. Ein weiterer Antikörper des erfindungsgemäßen Sandwichtests kann entweder ebenfalls ein MAK oder aber ein polyklonaler Antikörper (PAK; im Englischen: polyclonal antibody oder PAB) sein.

Bevorzugt ist eine Antikörperkombination umfassend mindestens einen polyklonalen Antikörper (PAK; im Englischen polyclonal antibody oder PAB) und einen monoklonalen Antikörper (MAK; im Englischen momoclonal antibody oder MAB) (sogenannte PAK/MAK Kombination) gegen NT-proBNP.

Im Sprachgebrauch dieser Patentanmeldung bedeutet PAK (X-Y) einen polyklonalen Antikörper, der gegen das Epitop von NT-proBNP mit den Aminosäuren X bis Y gerichtet ist. MAK (X-Y) ist ein entsprechender monoklonaler Antikörper. AK (X-Y) bezeichnet generell einen Antikörper (z. B. PAK oder MAK), der gegen das Epitop von NT-proBNP mit den Aminosäuren X bis Y gerichtet ist.

MAK a.b.c. (X-Y) ist ein aus der hinterlegten Zelllinie a.b.c. gewonnener, gegen das Epitop von NT-proBNP mit den Aminosäuren X bis Y gerichteter monoklonaler Antikörper.

Um eine reproduzierbare Qualität des Antikörper-Label-Konjugates zu garantieren wird bevorzugt der MAK auf dem partikulären Label, insbesondere auf einem Goldlabel, immobilisiert. Weitere geeignete partikuläre Label sind beispielsweise gefärbte Latices, andere Metallsollabel, Polymerlabel, oder Halbleiter-Nanokristalle (sogenannte Quantum Dots). Vorzugsweise wird das MAK-Label-Konjugat so auf dem Schnelltestdevice zur Verfügung gestellt, dass es durch die Probenflüssigkeit von diesem abgelöst werden kann, beispielsweise durch Imprägnierung geeigneter Trägermaterialien wie Vliese, Membranen etc. Es ist jedoch auch möglich, das MAK-Label-Konjugat als Lösung zum Schnelltest zuzugeben.

Der PAK, der vorzugsweise durch Immunisieren von Säugetieren, insbesondere Schafen, Ziegen, oder Kaninchen gewonnen wird, wird vorzugsweise als Biotinderivat im Schnelltest angeboten und kann an einen Avidin- oder Streptavidin-Nachweisstrich gebunden werden. Es ist jedoch auch möglich, den PAK direkt im Schnelltestdevice zu immobilisieren, beispielsweise in Form eines Nachweisstriches auf einer geeigneten Chromatographiemembran.

Der erfindungsgemäß eingesetzte MAK muss nicht unbedingt das im Referenzsystem (Elecsys® Test) erkannte Epitop (AS 1-21) erkennen, um eine gute Korrelation mit dem Referenztest zu gewährleisten: Eine gute Korrelation mit dem Elecsys®-Referenzsystem, welches polyklonale Antikörper gegen die Epitope AS1-21 und AS39-50 von NT-proBNP verwendet (PAK (1-21) und PAK (39-50)), zeigen die in Anspruch 1 genannten Antikörperkombinationen, insbesondere die MAK/PAK-Kombination MAK 18.4.34(27-31)/PAK (39-50).

Die polyklonalen Antikörper, beispielsweise PAK (39-50) können nach an sich dem Fachmann bekannten Verfahren gewonnen, charakterisiert und identifiziert werden, insbesondere in Analogie zu Beispiel 2 von WO 00/45176.

Die monoklonalen Antikörper, beispielsweise MAK (38-42) und MAK (44-50) können nach an sich dem Fachmann bekannten Verfahren gewonnen, charakterisiert und identifiziert werden, insbesondere in Analogie zu Beispiel 3 von WO 00/45176 oder Beispiel 3 der EP-Patentanmeldung Nr. 03 010 591.0 vom 12.5.2003 (Klemt et al.).

Die Markierung der Antikörper, beispielsweise mit Gold- oder anderen Labeln, Biotin etc. erfolgt nach an sich dem Fachmann bekannten Methoden (vgl. auch hierzu Beispiel 2 in WO 00/45176 und Beispiel 2 in der EP-Patentanmeldung Nr. 03 010 591.0 vom 12.5.2003 von Klemt et al.) Die Markierung mit Gold ist beispielsweise in EP-A 0 898 170 detailliert beschreiben.

Insbesondere die bevorzugten monoklonalen Antikörper MAK 16.1.39 (38-42), MAK 18.29.23 (64-67) und MAK 18.4.34(27-31) sind gemäß Beispiel 3 der EP-Patentanmeldung Nr. 03 010 591.0 vom 12.5.2003 von Klemt et al. zu gewinnen. Entsprechende Zelllinien sind bei der Deutschen Sammlung von Mikroorganismen und Zellkulturen GmbH (DSZM) hinterlegt (Eingangsnummern der Hinterlegungsstelle und Datum der Hinterlegung: DSM ACC2590 (7. Mai 2003) für MAK 16.1.39 (38-42); DSM ACC2593 (7. Mai 2003) für MAK 18.29.23 (64-67); und DSM ACC2592 (7. Mai 2003) für MAK 18.4.34(27-31).

Die Kombination MAK 18.4.34(27-31) / PAK (39-50) führt zu einer vergleichbar guten Korrelation mit dem Referenztest wie die Kombination MAK 17.3.1(13-16) / PAK (39-50) nicht Teil der beanspruchten Erfindung, siehe auch Beispiel 2).

Für den erfindungsgemäßen Test erwiesen sich zudem die Kombination MAK 18.4.34(27-31) / PAK (39-50) als besonders vorteilhaft: Mit dieser Kombination konnte eine besonders für Schnellteste geeignete Funktionskurve angepasst werden (siehe Beispiel 3). Die nicht erfindungsgemäße Kombination MAK 17.3.1(13-16) / PAK (39-50) zeigte im Vergleich dazu eine schlechtere Empfindlichkeit des Testes.

Die Erfindung wird anhand der nachfolgenden Beispiele und Figuren näher erläutert.

In Figur 1 ist schematisch eine bevorzugte Ausführungsform eines erfindungsgemäßen Schnelltestdevices in Form eines immunochromatographischen Teststreifens abgebildet.
Figur 2 zeigt die Korrelation der Antikörper-Kombination MAK 17.3.1(13-16) / PAK (39-50) im Elecsys®-Nasstestformat mit der Elecsys®-Referenzmethode PAK (1-21)/PAK (39-50).
Figur 3 zeigt die Korrelation der Antikörper-Kombination MAK 18.4.34 (27-31) / PAK (39-50) im Elecsys®-Nasstestformat mit der Elecsys®-Referenzmethode PAK (1-21)/PAK (39-50).
Figur 4 zeigt Funktionskurven von NT-proBNP-Teststreifen gemäß Beispiel 1 mit unterschiedlichen Antikörperkombinationen.
Figur 5 zeigt die Korrelation eines NT-proBNP-Testreifens mit der Antikörperkombination: Au-MAK18.4.34(27-31) / Bi-PAK (39-50) zum Elecsys® NT-proBNP-Testkit.

Die Ziffern in den Figuren bedeuten:
- 1: Probenaufgabezone
- 2: Erythrozytenabtrennzone
- 3: Detektionszone
- 4: Saugzone
- 5: Trägermaterial
- 6: Probenaufgabematrix ("Biotinvlies" und "Goldvlies")
- 7: Erythrozytenabtrennmatrix
- 8: Detektionsmatrix
- 9: strichförmige Immobilisierungszone
- 10: Saugmatrix

### Beispiele

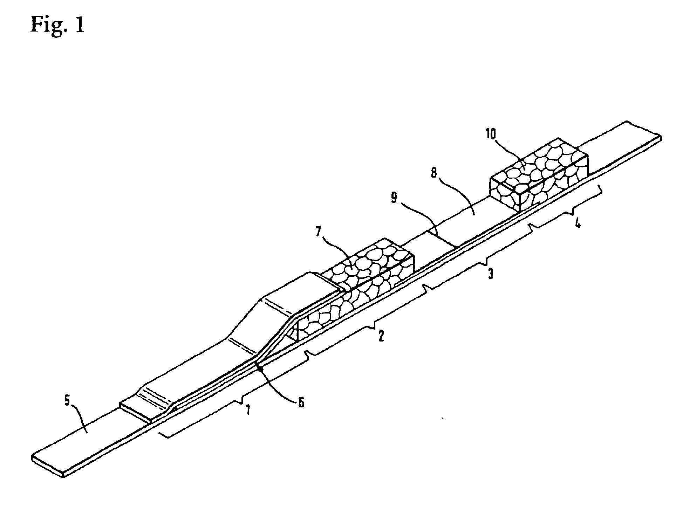

### 1) Herstellung eines Testdevices zur Bestimmung von NT-proBNP aus Volblut (vgl. Fig. 1)

Das Testdevice (Fig. 1) besteht aus einem Trägermaterial (5) auf das eine Probenaufgabezone (1), eine Erythrozytenabtrennzone (2), eine Detektionszone (3) und eine Saugzone (4) aufgebracht sind. In der Probenaufgabezone (1) ist eine Probenaufgabematrix (6) angeordnet, die eine Erythrozytenabtrennmatrix (7) teilweise überlappt. Die Erythrozytenabtrennmatrix (7) ihrerseits überlappt etwas die Detektionsmatrix (8) (Detektionszone), auf der eine immobilisierte Substanz in Form eines Striches (9) aufgebracht ist. Eine Saugmatrix (10) überlappt etwas die Detektionsmatrix (8). In der Probenaufgabematrix (6) sind alle diejenigen Reagenzien untergebracht, die zur Bildung eines Komplexes mit dem nachzuweisenden Analyten erforderlich sind. Im vorliegenden Fall besteht die Probenaufgabezone aus 2 übereinanderliegenden Vliesen, wobei das erste ("Goldvlies") mit einem goldmarkierten Antikörper gegen NT-proBNP (MAK 18.4.34 (27-31)) getränkt ist und das zweite Vlies ("Biotinvlies") einen biotinylierten Antikörper gegen NT-proBNP (PAK (39-50)) enthält. Innerhalb der Detektionszone ist ein Strich (9) aus Streptavidin aufgebracht.

Als Trägerschicht 5 wird eine 350 µm dicke Polyesterfolie (Pütz) verwendet. Als "Goldvlies" bzw. "Biotinvlies" der Probenaufgabematrix 6 wird ein 360 µm dickes Polyestervlies (Roche Diagnostics) verwendet. Als Erytrozytenabtrennmatrix 7 wird ein 1.8 mm dickes Glasfaservlies (Roche Diagnostics) verwendet. Als Detektionsmatrix 8 dient eine 140 µm dicke Nitrocellulosemembran (Sartorius). Als Saugmatrix 10 dient ein 1.8 mm dickes Glasfaservlies (Roche Diagnostics). Die einzelnen Komponenten (6, 7, 8, 10) werden wie in Fig. 1 gezeigt mittels Schmelzkleber leicht überlappend auf die Trägerschicht 5 aufgeklebt.

### Die Tränkrezeptur für die "Gold- und Biotinvliese" ist:

- Biotinvlies":: 100 mM Hepes pH 7.4, 0.1 % Tween®,
20 µg/mL biotinylierter PAK (39-50)
- "Goldvlies":: 100 mM Hepes pH 7.4, OD 4 MAK 18.4.34 (27-31)-Goldkonjugat

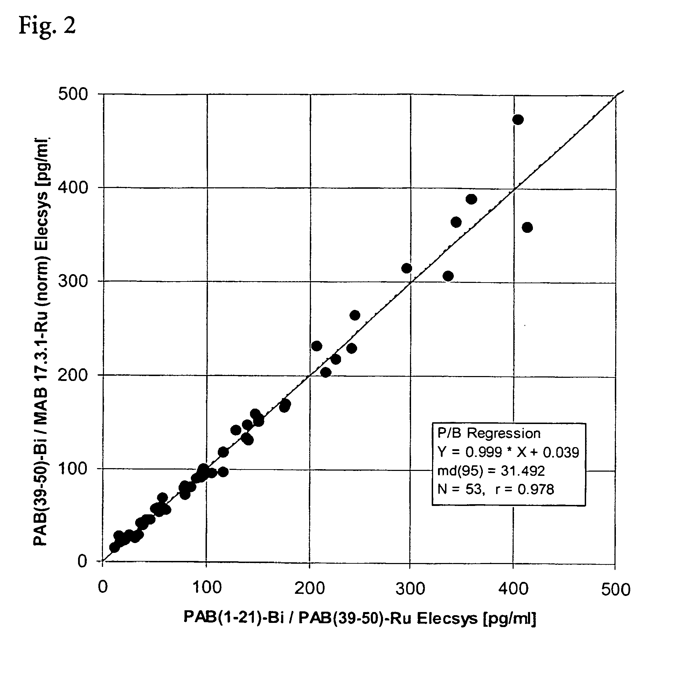

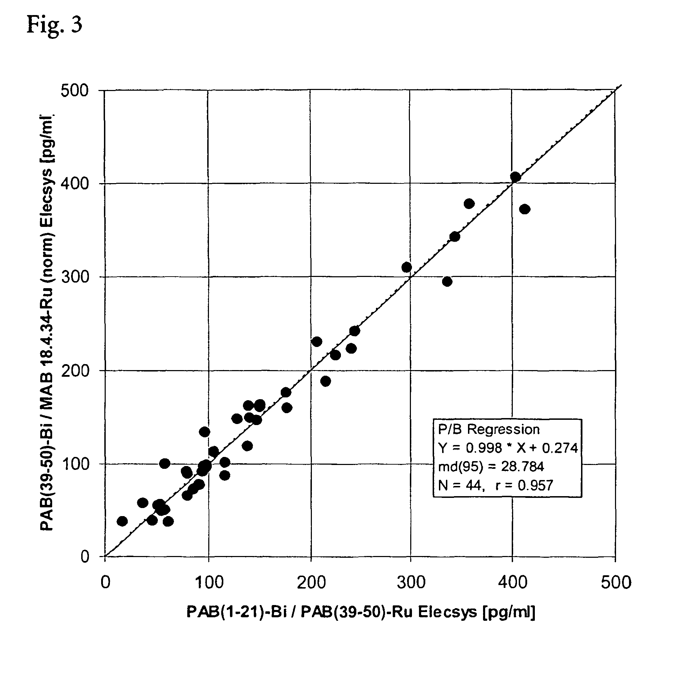

### 2) Korrelation der Epitop / Antikörper Kombinationen MAK 17.3.1 (13-16) / PAK (39-50) und MAK 18.4.34(27-31) / PAK (39-50) im Elecsys® Format zum Elecsys® NT-proBNP-Testkit (vgl. Fig. 2 und 3)

Die Korrelation der MAK / PAK Kombinationen MAK 17.3.1(13-16) / PAK (39-50) und MAK 18.4.34(27-31) / PAK (39-50) zum Elecsys®-Testkit (PAK (1-21)/PAK (30-50)) wurde in einem Elektrochemilumineszenz Immunoassay auf Elecsys® 2010 (Roche Diagnostics) untersucht. Dazu wurde der PAK (39-50) als biotinyliertes Fangreagenz und ruthenilierte F(ab')₂ Fragmente der MAKs als Detektionsreagenz eingesetzt. 20 µl Probe oder Standard Material wurde mit jeweils 75 µl der beiden Antikörper Reagenzien für 9 Minuten bei 37 C inkubiert. Danach wurden 35 µl Streptavidin beschichtete magnetische Polystyrol Partikel zugegeben und für weitere 9 Minuten bei Raumtemperatur inkubiert. Das Elektrolumineszenz Signal eines Aliquots der Inkubationslösung wurde standardmässig auf Elecsys® 2010 gemessen und über eine Standardkurve in ein Konzentrationssignal umgerechnet.

Klinische Proben von Herzinsuffizienzpatienten wurden nun mit beiden MAK/PAK Testvarianten und dem Elecsys® Kit vermessen. Die Ergebnisse sind in den Figuren 2 und 3 abgebildet. Mit beiden MAK/PAK Varianten erhält man eine sehr gute Korrelation zum Elecsys® Kit (r=0.978 und r=0.957).

### 3) Funktionskurve eines NT-proBNP-Testtreifens mit zwei unterschiedlichen MAK/PAK-Kombinationen

Ein NT-proBNP-Teststreifen wurden gemäss Beispiel 1 hergestellt. Dabei wurde folgende Tränkrezeptur für die Reagenzvliese verwendet:
- "Biotinvlies":: 100 mM Hepes pH 7.4, 0.1 % Tween®
20 µg/mL biotinylierter PAK (39-50)
- "Goldvlies":: 100 mM Hepes pH 7.4, OD 4 MAK 18.4.34 (27-31)- oder MAK 17.3.1 (13-16)-Goldkonjugat

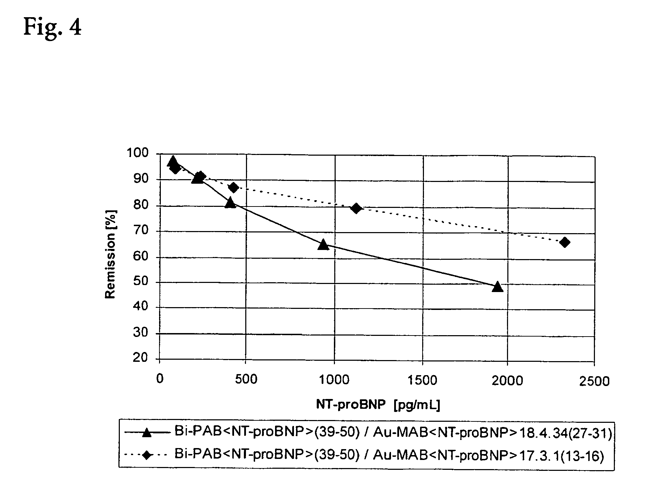

Heparinisierte Blutproben von gesunden Spendern wurden mit NT-proBNP-haltigen Seren von Herzinsuffizienzpatienten aufgestockt und aliqotiert. 150 µl der aufgestockten Blutproben wurden auf den Teststreifen pipettiert und auf dem CARDIAC Reader® (Roche Diagnostics) vermessen. Die Reaktionszeit nach der Probenerkennung betrug 12 Minuten. Zur Bestimmung der NT-proBNP-Konzentration der Proben wurde von einem Aliquot das Plasma abzentrifugiert und mit einem Elecsys® NT-proBNP-Kit (Roche Diagnostics) vermessen. Die so erhaltenen Funktionskurven der beiden Teststreifenvarianten MAK 17.3.1(13-16) / PAK (39-50) und MAK 18.4.34(27-31) / PAK (39-50) sind in Figur 4 abgebildet. Für die Variante MAK 18.4.34(27-31) /PAK (39-50) ergibt sich eine deutlich steilere Standardkurve und damit ein sensitiverer Test.

### 4) Korrelation eines NT-proBNP-Testreifens mit der AK Kombination: Au-MAK18.4.34(27-31) / Bi-PAK (39-50) zum Elecsys® NT-proBNP-Testkit

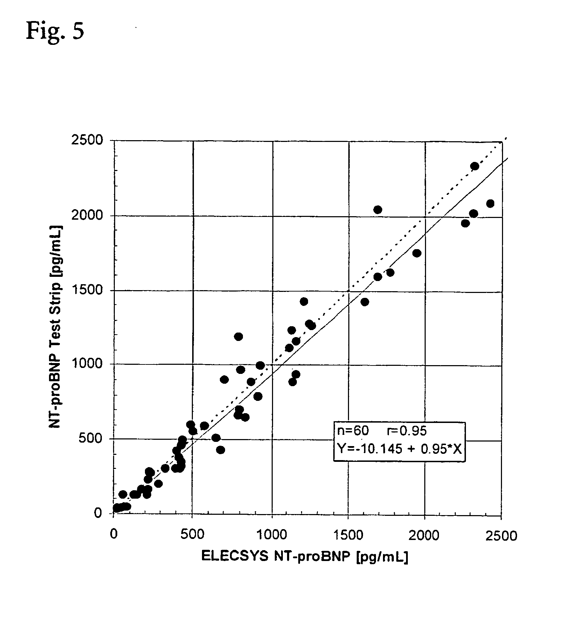

Heparinisierte Blutproben gesunder Spender wurden mit NT-proBNP-haltigen Seren von Herzinsuffizienzpatienten versetzt und aliquotiert. 150 µl dieser "aufgestockten" Blutproben wurden auf den Teststreifen pipettiert und im CARDIAC Reader® (Roche Diagnostics) nach dem Standardverfahren vermessen. Von der gleichen Probe wurde Plasma abzentrifugiert und mit dem Elecsys® NT-proBNP-Kit auf einem Elecsys® 1010 Analysensytem (Roche Diagnostics) vermessen. Auf diese Weise wurden 60 Proben hergestellt und mit beiden Systemen gemessen. Figur 5 zeigt die Messwerte für beide Systeme. Die Korrelation ist mit r=0.95 sehr gut.

## Patentansprüche

1. Testelement in Form eines nach dem Sandwich-Prinzip arbeitenden immunographischen Teststreifens, **dadurch gekennzeichnet, dass**
das Testelement mindestens zwei Antikörper gegen NT-proBNP umfasst,
wobei mindestens einer dieser Antikörper ein MAK ist,
und die Antikörper in folgenden Antikörperkombinationen vorliegen:
MAK 18.4.34 (27-31) mit: PAK (39-50) oder PAK (38-42) oder PAK (44-50);
oder
MAK 18.4.34 (27-31) mit MAK (38-42); oder MAK 18.9.8 (27-31) mit MAK (38-42),
und einer der Antikörper als Konjugat mit einem partikulären Label vorliegt.

2. Testelement gemäß Anspruch 1, **dadurch gekennzeichnet, dass** MAK (38-42) der MAK 16.1.39 (38-42) ist.

3. Testelement gemäß Anspruch 1 oder 2, **dadurch gekennzeichnet, dass** einer der Antikörper als Antikörper-Gold-Konjugat vorliegt.

4. Testelement gemäß Anspruch 1 oder 2, **dadurch gekennzeichnet, dass** einer der Antikörper als Konjugat mit einem gefärbten Latex-Partikel vorliegt.

5. Verfahren zum Nachweis von NT-proBNP, **dadurch gekennzeichnet, dass** ein Testelement gemäß einem der Ansprüche 1 bis 4 verwendet wird.

## Claims

1. Test element in the form of an immunographic test strip operating according to the sandwich principle, **characterized in that**
the test element comprises at least two antibodies against NT-proBNP, wherein at least one of these antibodies is a monoclonal antibody and the antibodies are present in the following antibody combinations:
MAB 18.4.34 (27-31) with PAB (39-50) or PAB (38-42) or PAB (44-50);
or
MAB 18.4.34 (27-31) with MAB (38-42); or MAB 18.9.8 (27-31) with MAB (38-42),
and one of the antibodies is present as a conjugate with a particulate label.

2. Test element according to claim 1, **characterized in that** MAB (38-42) is the MAB 16.1.39(38-42).

3. Test element according to claim 1 or 2, **characterized in that** one of the antibodies is present as an antibody-gold conjugate.

4. Test element according to claim 1 or 2, **characterized in that** one of the antibodies is present as a conjugate with a coloured latex particle.

5. Method for the detection of NT-proBNP, **characterized in that** a test element according to one of the claims 1 to 4 is used.

## Revendications

1. Élément de test sous la forme d'une bandelette réactive immunographique travaillant conformément au principe de l'essai sandwich, **caractérisé en ce que**
l'élément de test comprend au moins deux anticorps dirigés contre NT-proBNP,
au moins un de ces anticorps étant un MAK,
et les anticorps étant présents dans les combinaisons d'anticorps ci-après :
MAK 18.4.34 (27-31) avec : PAK (39-50) ou PAK (38-42) ou PAK (44-50) ; ou
MAK 18.4.34 (27-31) avec MAK (38-42) ; ou MAK 18.9.8 (27-31) avec MAK (38-42),
et un des anticorps étant présent sous la forme d'un conjugué avec un marqueur particulaire.

2. Élément de test selon la revendication 1, **caractérisé en ce que** MAK (38-42) est le MAK 16.1.39 (38-42).

3. Élément de test selon la revendication 1 ou 2, **caractérisé en ce qu'**un des anticorps est présent sous la forme d'un conjugué anticorps-or.

4. Élément de test selon la revendication 1 ou 2, **caractérisé en ce qu'**un des anticorps est présent sous la forme d'un conjugué avec une particule de latex colorée.

5. Procédé pour le décèlement de NT-proBNP, **caractérisé en ce qu'**on utilise un élément de test selon l'une quelconque des revendications 1 à 4.
